# EUROPÄISCHE PATENTSCHRIFT

(11) **EP 2 978 864 B1**
(45) Veröffentlichungstag und Bekanntmachung des Hinweises auf die Patenterteilung: **17.10.2018**
(21) Anmeldenummer: 14723670.7
(22) Anmeldetag: 25.03.2014
(51) Int. Cl.: G01N 7/18

(54) **LANZE UND VERFAHREN ZUR BESTIMMUNG VON REAKTIONSDATEN EINES REAKTIONSABLAUFS**
LANCE AND METHOD FOR DETERMINING REACTION DATA OF THE COURSE OF A REACTION
LANCE ET PROCÉDÉ PERMETTANT DE DÉTERMINER DES DONNÉES DE RÉACTION RENSEIGNANT SUR LA PROGRESSION D'UNE RÉACTION

(30) Priorität: 25.03.2013 AT 502042013
(43) Veröffentlichungstag der Anmeldung: 03.02.2016
(73) Patentinhaber: Voestalpine Stahl GmbH, 4020 Linz (AT)
(72) Erfinder: SCHUSTER, Stefan, A-4470 Enns (AT); LENGAUER, Gunter, A-4210 Gallneukirchen (AT); PANHOFER, Harald, A-4030 Linz (AT)
(74) Vertreter: Jell, Friedrich
(86) Internationale Anmeldenummer: PCT/AT2014/050074
(87) Internationale Veröffentlichungsnummer: WO 2014/153585

(56) Entgegenhaltungen:
- DE-A1- 2 239 216
- DE-A1- 2 546 647
- GB-A- 934 112
- US-A- 5 443 572
- US-A1- 2002 180 124

## Beschreibung

### Technisches Gebiet

Die Erfindung betrifft eine Lanze und ein Verfahren zur Bestimmung von Reaktionsdaten eines Reaktionsablaufs, bei dem ein Reaktionsgas mit Hilfe mindestens einer Lanze auf eine metallische Schmelze in einem metallurgischen Gefäß aufgeblasen wird und dabei Messdaten erfasst werden, in deren Abhängigkeit Reaktionsdaten zum Reaktionsablauf bestimmt werden, wobei die Lanze zur Erfassung von Messdaten über mindestens eine Mündungsöffnung wenigstens einer Messleitung ein gegenüber dem Reaktionsgas getrennt geführtes Gas ausbläst.

### Stand der Technik

Um den Reaktionsablauf eines Aufblasverfahrens bzw. Sauerstoff-Aufblasverfahrens überwachen bzw. steuern zu können, ist es aus dem Stand der Technik bekannt (DE1290557A1), die elektrische Leitfähigkeit zwischen der Lanze und dem metallurgischen Gefäß während des Reaktionsablaufs zu messen. Mithilfe dieser erfassten Messdaten wird der Reaktionsablauf interpretiert bzw. werden damit Reaktionsdaten zum Reaktionsablauf bestimmt. Insbesondere soll damit auch ein Überschäumen der Schaumschlacke, die sich beim Aufblasen von Sauerstoff mit einer hohen Strömungsgeschwindigkeit auf die Schmelze im metallurgischen Gefäß ausbildet, erkannt und in weiterer Folge die Gefahr eines Auswurfs vermindert werden. Allerdings ist die gemessene elektrische Leitfähigkeit von einer Vielzahl von Reaktionsparametern des Reaktionsablaufs abhängig, weshalb die Gefahr eines Überschäumens mithilfe derartiger Verfahren nur schwer zuverlässig erkannt werden kann. Des Weiteren bilden sich metallurgische Veränderungen im Reaktionsablauf in der elektrischen Leitfähigkeit vergleichsweise träge ab, was eine dynamische Bestimmung von Reaktionsdaten zum Reaktionsablauf erschwert.

Außerdem ist aus der DE2239216A1 ein Verfahren zur Ermittlung der Eintauchtiefe einer Lanze in einer metallischen Schmelze bekannt. Hierzu wird über eine Messleitung der Lanze ein gegenüber dem Reaktionsgas der Lanze getrennt geführtes Druckgas am unteren Ende der Lanze ausgeblasen. Messdaten zu den Druckunterschieden zwischen einem Umgebungsdruck und dem Druck am unteren Ende der Lanze dienen der Ermittlung der Eintrauchtiefe der Lanze. Nachteilig ist während der Messung mit äußerst heftigen chemischen Reaktionen des Reaktionsgases mit der Schmelze zu rechnen, was die Messdatenerfassung verfälscht und damit die Standfestigkeit des Verfahrens gefährden kann.

Zudem ist es zur Messung der Höhe einer Schaumschlacke in einem metallurgischen Gefäß bekannt (US4359211 B), durch die Wand des metallurgischen Gefäßes auf unterschiedlichen Höhen Druckgas einzudüsen. Eine derartige Messeinrichtung bedarf einer vergleichsweise aufwendigen konstruktiven Änderungen am metallurgischen Gefäß, um trotz Wandöffnungen stets dessen Dichtheit gegenüber einem Austritt von metallurgischer Schmelze gewährleisten zu können.

### Darstellung der Erfindung

Die Erfindung hat sich daher die Aufgabe gestellt, ausgehend vom eingangs geschilderten Stand der Technik ein Verfahren zu schaffen, das Reaktionsdaten zu einem metallurgischen Reaktionsablauf schnell und zuverlässig bestimmen kann. Zudem soll das Verfahren einfach und standfest gegenüber metallurgischen Prozessen sein.

Die Erfindung löst die gestellte Aufgabe dadurch, dass die Lanze zur Erfassung von Messdaten über mindestens eine Mündungsöffnung wenigstens einer Messleitung das gegenüber dem Reaktionsgas getrennt geführte Gas seitlich ausbläst, von dem der Innendruck wenigstens einer sich an dieser Mündungsöffnung der jeweiligen Messleitung ausbildenden Gasblase gemessen wird, wie im Ansprüche 1 und 9 offenbart.

Bläst die Lanze zur Erfassung von Messdaten über mindestens eine Mündungsöffnung wenigstens einer Messleitung das gegenüber dem Reaktionsgas getrennt geführte Gas seitlich aus, von dem der Innendruck wenigstens einer sich an dieser Mündungsöffnung der jeweiligen Messleitung ausbildenden Gasblase gemessen wird, können Messdaten schnell und zuverlässig erfasst werden. In deren Abhängigkeit können in weiterer Folge Reaktionsdaten zum Reaktionsablauf bestimmt werden. Eine Druckmessung an der Messleitung mit einer längsseitlich der Lanze ausmündenden Mündungsöffnung ist nämlich verhältnismäßig einfach durchzuführen und kann aufgrund des Umstands einer direkten Interaktion der Gasblase mit der Schmelze bzw. mit deren Schaumschlacke auch äußerst reaktionsschnell erfolgen, was zahlreiche Vorteile - auch hinsichtlich der Verfahrenssicherheit - nach sich ziehen kann. Zudem kann solch eine Druckmessung ausreichend weit von den durch den Reaktionsablauf bedingten Belastungen beabstandet durchgeführt werden, was ein äußerst standfestes und genaues Verfahren mit reproduzierbaren Ergebnissen ermöglichen kann.

Im Allgemeinen wird erwähnt, dass sich als Reaktionsgas Sauerstoff auszeichnen kann.

Um die Messgenauigkeit der Reaktionsdaten weiter zu erhöhen, kann vorgesehen sein, dass die Lanze über mindestens zwei Messleitungen Gas seitlich ausbläst, wobei in Abhängigkeit einer Differenzbildung der gemessenen Innendrücke der Gasblasen Reaktionsdaten zum Reaktionsablauf bestimmt werden. Mithilfe dieser Differenzbildung können selbst geringe Änderungen im metallurgischen Reaktionsablauf gemessen werden, sodass metallurgische Vorgänge mithilfe des erfindungsgemäßen Verfahrens besonders rasch erfasst werden können.

Bilden die auf gleicher Lanzenhöhe ausmündenden Messleitungen voneinander unterschiedlich große Gasblasen aus, können damit nicht nur für eine Differenzbestimmung geeignete Druckmessdaten erfasst werden, zusätzlich können diese Druckmessdaten auch zur Erhöhung der Genauigkeit der Reaktionsdaten beitragen. Aufgrund der auf gleicher Lanzenhöhe ausmündenden Messleitungen ist es nämlich unter anderem möglich, auf beide Messungen wirkende Störungen zu unterdrücken und Messdaten mit erhöhtem Rauschabstand zu erzeugen.

Es ist aber auch vorstellbar, dass auf unterschiedlichen Lanzenhöhen ausmündende Messleitungen gleich große Gasblasen ausbilden. Insbesondere kann durch Austragung von Gasblasen auf unterschiedlichen Lanzenhöhen verfahrenssicher auf eine Füllstandhöhe der Schaumschlacke im Gefäß rückgeschlossen werden.

Eine erhöhte Messgenauigkeit kann sich ergeben, wenn auf unterschiedlichen Lanzenhöhen ausmündende Messleitungen unterschiedlich große Gasblasen ausbilden.

Die gegenseitige Beeinflussung der Messleitungen kann erheblich verringert werden, wenn die Messleitungen an gegenüberliegenden Seiten am Lanzenkörper Gas ausblasen. Mit einer weiter erhöhten Messgenauigkeit kann auf diese Weise gerechnet werden. Besonders kann sich hierbei auszeichnen, wenn die Messleitungen am Lanzenkörper diametral gegenüberliegend Gas ausblasen.

Wird die Messleitung bzw. werden die Messleitungen von dem die Lanze kühlenden Kühlmedium mitgekühlt, kann für gleichbleibende Bedingungen bei der Gasaustragung gesorgt werden. Eine verbesserte Reproduzierbarkeit des Verfahrens kann sich auf diese Weise einstellen.

Besonders auszeichnen kann sich das erfindungsgemäße Verfahren, indem damit Reaktionsdaten bestimmt werden, anhand welcher der Reaktionsablauf eines Aufblasverfahrens gesteuert oder geregelt wird. Damit kann es beispielsweise ermöglicht werden, rechtzeitig einen Auswurf zu erkennen und so die Gefahr eines Auswurfs zu reduzieren bzw. diesbezügliche Gegenmaßnahmen einzuleiten.

Die Erfindung hat sich weiter die Aufgabe gestellt, eine konstruktiv einfache und robuste Lanze zum Aufblasen eines Reaktionsgases auf eine in einem metallurgischen Gefäß befindliche metallische Schmelze zu schaffen, mit der schnell und reproduzierbar Messdaten gewonnen werden können, um Reaktionsdaten zum Reaktionsablauf bestimmten zu können.

Die Erfindung löst die Aufgabe hinsichtlich der Lanze dadurch, dass die Messleitung in mindestens einer Mündungsöffnung seitlich am Lanzenmantel des Lanzenkörpers endet und zur Erzeugung mindestens einer Gasblase ausgebildet ist.

Endet die Messleitung in mindestens einer Mündungsöffnung seitlich am Lanzenmantel des Lanzenkörpers, kann diese am Lanzenmantel längsseitige Öffnung genutzt werden, vergleichsweise weit vom Aufblasbereich entfernt Messdaten zu erfassen. Die Messung kann damit vergleichsweise unempfindlich gegenüber den in diesem untereren Bereich der Lanze auftretenden turbulenten Strömungen durchgeführt werden. Insbesondere wenn diese Mündungsöffnung zur Erzeugung mindestens einer Gasblase ausgebildet ist und es einer vergleichsweise störungsfreien Zone zur Einperlung von Gasblasen in die Schmelze oder Schaumschlacke bedarf, um rasche und genaue Messdaten zum Reaktionsverlauf erfassen zu können. Damit kann auch mithilfe der erfindungsgemäßen Lanze beispielsweise die Gefahr eines unerwünschten Auswurfs vermindert bzw. können damit rechtzeitig geeignete Gegenmaßnahmen eingeleitet werden. Zudem ist damit eine zuverlässige Früherkennung eines nachteiligen Auswurfs möglich.

Vorteilhaft können diese Messdaten über die Lanze vergleichsweise weit von durch den Reaktionsablauf verursachten Belastungen beabstandet ermittelt werden, wenn eine Messeinrichtung vorgesehen ist, die einen Sensor zur Erfassung von Messdaten aufweist, der mit der Messleitung zur Erfassung von, vom Innendruck der Gasblase abhängigen Messdaten verbunden ist.

Eine Differenzmessung kann ermöglicht werden, wenn der Lanzenkörper mindestens zwei Messleitungen aufweist, die seitlich am Lanzenkörper ausmünden.

Für solch eine Differenzmessung kann ausreichend sein, wenn die Mündungsöffnungen der Messleitungen unterschiedliche Mündungsquerschnitte aufweisen. Hierzu können selbst die Messleitungen auf gleicher Hohe am Lanzenkörper ausmünden.

Alternativ ist auch denkbar, dass die Mündungsöffnungen der Messleitungen auf unterschiedlicher Höhe am Lanzenmantel seitlich ausmünden. Zudem kann dies in Kombination mit unterschiedlichen Mündungsquerschnitten für eine erhöhte Empfindlichkeit sorgen. Die Genauigkeit der Messdaten kann damit erhöht werden.

Die gegenseitige Beeinflussung der Messleitungen kann verringert werden, wenn ihre Mündungsöffnungen an gegenüberliegenden Seiten am Lanzenkörper ausmünden. Damit kann nicht nur die Messgenauigkeit weiter erhöht werden, es kann sich damit auch der Konstruktionsaufwand, insbesondere in Bezug auf die Kühlung, der Lanze verringern. Besonders kann sich hierfür auszeichnen, wenn die Mündungsöffnungen am Lanzenkörper diametral gegenüberliegend ausmünden.

Die Temperatur an der Ausperlöffnung der Messleitung kann stabilisiert werden, wenn die Messleitung mit dem gekühlten Lanzenmantel des Lanzenkörpers zumindest bereichsweise thermisch leitend verbunden ist. Eine erhöhte Messgenauigkeit kann damit erreicht werden.

### Kurze Beschreibung der Zeichnung

In den Figuren ist beispielsweise der Erfindungsgegenstand anhand einer Ausführungsvariante näher dargestellt. Es zeigen
- Fig. 1: eine aufgerissene Seitenansicht auf eine Vorrichtung zur Bestimmung von Reaktionsdaten und
- Fig. 2: eine Detailansicht der Fig. 1.

### Weg zur Ausführung der Erfindung

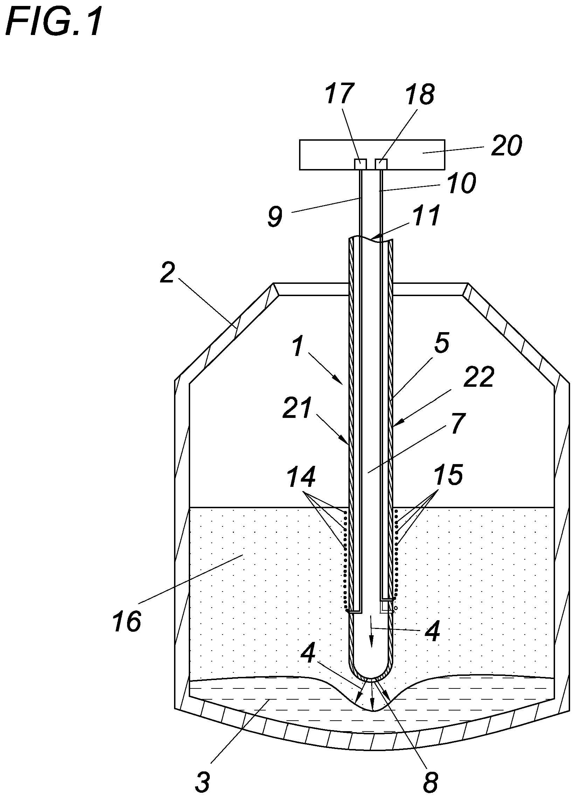

Gemäß Fig. 1 ist eine Lanze 1 dargestellt, die in ein metallurgisches Gefäß 2 eintaucht, das eine metallische Schmelze 3, beispielsweise einen Eisenwerkstoff, einen Aluminiumwerkstoff, eine metallische Legierung oder dergleichen, aufweist. Die Lanze 1 wird dazu verwendet, ein Reaktionsgas 4 auf die Schmelze 3 aufzublasen, um damit einen metallurgischen Reaktionsablauf in Gang zu setzen. Solch ein Reaktionsablauf kann etwa ein Frischen von Roheisen mit Sauerstoff sein, was durch das LD-Verfahren bekannt ist. Um den Belastungen, die durch den Reaktionsablauf verursacht werden, standhalten zu können, ist die Lanze 1 mit einem gekühlten äußeren Lanzenmantel 5 versehen. Solch eine Kühlung kann beispielsweise durch eine doppelwandige Ausführung des Lanzenmantels 5 geschaffen werden, der Wasser als Kühlmittel führt, was in den Zeichnungen der Übersichtlichkeit wegen jedoch nicht weiter dargestellt ist. Die Gasführung 7 des Reaktionsgases 4 der Lanze 1 mündet im unteren Ende der Lanze 1 über eine Öffnung 8 oder nicht näher dargestellt mehrere Öffnungen des Lanzenmantels 5 aus und strömt auf die Schmelze 3 auf. Es braucht nicht weiter erwähnt werden, dass jegliche Öffnungsform bzw. Anzahl an Öffnungen 8 denkbar ist.

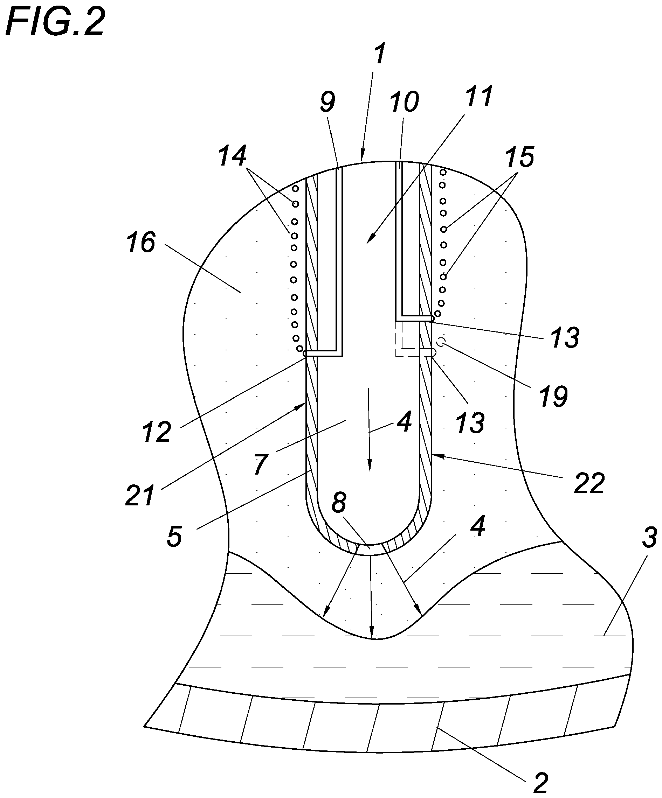

Die Lanze 1 weist mindestens eine - im gegenständlichen Ausführungsbeispiel zwei Messleitungen 9 bzw. 10 - auf, die im Lanzenkörper 11 geschützt vor den Einflüssen des Aufblasverfahrens vorgesehen ist. Diese Messleitungen 9 bzw. 10 mündet erfindungsgemäß in mindestens einer Mündungsöffnung. Im Beispiel sind zwei Mündungsöffnungen 12 bzw. 13 am seitlichen bzw. längsseitigen Lanzenmantel 5 des Lanzenkörpers 11 vorgesehen. Die Mündungsöffnungen 12 bzw. 13 - als Ausperlöffnung ausgeführt - erzeugen Gasblasen 14 bzw. 15, was der Fig. 2 besser entnommen werden kann. Diese Gasblasen 14 bzw. 15 perlen in die Schaumschlacke 16 ein, die sich oberhalb der Schmelze 3 infolge des metallurgischen Reaktionsablaufs ausbildet. Mit einer Messeinrichtung 20, die mit Sensoren 17 bzw. 18 Messdaten zum jeweiligen Druck der Messleitungen 9 bzw. 10 und damit auch den Innendruck der Gasblasen 14 bzw. 15 an der jeweiligen Mündungsöffnung 12 bzw. 13 erfasst, ist es nun möglich, genau und rasch auf Reaktionsdaten des Reaktionsablaufs zu schließen bzw. diese damit zu bestimmten. Ein bei Aufblasverfahren bekannter unerwünschter Auswurf kann so rechtzeitig erkannt werden - in weiterer Folge ist das Aufblasverfahren anhand der so erfassten Reaktionsdaten vorteilhaft regel- und steuerbar.

Da im Lanzenkörper 11 zwei Messleitungen 9, 10 vorgesehen sind, kann die Messung bzw. die Bestimmung der Reaktionsdaten über eine Differenzmessung ihrer Innendrücke erheblich genauer vorgenommen werden. Derartige Differenzmessungen sind beispielsweise von "Gas-Blasverfahren" aus dem Stand der Technik bekannt.

Um für eine ausreichend hohe Differenzbildung zu sorgen, enden die Mündungsöffnungen 12 und 13 der beiden Messleitung 9 und 10 auf unterschiedlicher Höhe seitlich am Lanzenmantel 5 aus.

Wie der Fig. 2 insbesondere zu entnehmen, ist es auch vorstellbar, dass diese Mündungsöffnungen 12, 13 auf gleicher Höhe am Lanzenmantel 5 seitlich ausmünden und unterschiedliche Mündungsquerschnitte aufweisen, um damit unterschiedlich große Gasblasen 14 und 19 erzeugen.

Es braucht nicht weiter erwähnt werden, dass eine Kombination aus unterschiedlichen Höhen der Mündungsöffnungen 12, 13 mit verschiedenen Mündungsquerschnitten vorstellbar ist. Dies ist jedoch nicht näher dargestellt.

Zudem sind die Messleitung 9 und 10 in ihren Mündungsbereichen mit dem gekühlten Lanzenmantel 5 thermisch leitend verbunden, um diese Messleitungen 9 und 10 mit dem Lanzenmantel 5 mitzukühlen und damit reproduzierbare Verfahrensbedingungen zur Einperlung von Gas 14, 15, 19 in die Schaumschlacke 16 zu gewährleisten.

Vorteilhaft ist weiter vorgesehen, dass die Mündungsöffnungen 12, 13 der Messleitungen 9, 10 an gegenüberliegenden Seiten 21, 22 am Lanzenkörper 11 ausmünden. Die Einperlung von Gas 14, 15, 19 ist damit in hohem Maße voneinander unabhängig - besonders dann, wenn die Mündungsöffnungen 12, 13 der Messleitungen 9, 10 am Lanzenkörper 11 diametral gegenüberliegen. Die Messgenauigkeit wird dadurch erheblich verbessert.

Im Allgemeinen wird erwähnt, dass - wie den Figuren 1 und 2 entnommen werden kann -, die Messleitungen 12, 13 jeweils an unterschiedlichen Seite 21, 22 bzw. Längsseiten des Lanzenkörpers 11 ausmünden, was jedoch nicht zwangsweise der Fall sein muss. Zudem ist auch hier eine Kombination aus unterschiedlichen Höhen der Mündungsöffnungen 12, 13 mit verschiedenen Mündungsquerschnitten vorstellbar ist - dies ist jedoch nicht näher dargestellt.

## Patentansprüche

1. Verfahren zur Bestimmung von Reaktionsdaten eines Reaktionsablaufs, bei dem ein Reaktionsgas (4) mit Hilfe mindestens einer Lanze (1) auf eine metallische Schmelze (3) in einem metallurgischen Gefäß (2) aufgeblasen wird und dabei Messdaten erfasst werden, in deren Abhängigkeit Reaktionsdaten zum Reaktionsablauf bestimmt werden, wobei die Lanze (1) zur Erfassung von Messdaten über mindestens eine Mündungsöffnung (12, 13) wenigstens einer Messleitung (9, 10) ein gegenüber dem Reaktionsgas (4) getrennt geführtes Gas ausbläst, **dadurch gekennzeichnet, dass** die Lanze (1) zur Erfassung von Messdaten über mindestens eine Mündungsöffnung (12, 13) wenigstens einer Messleitung (9, 10) das gegenüber dem Reaktionsgas (4) getrennt geführte Gas seitlich ausbläst, von dem der Innendruck wenigstens einer sich an dieser Mündungsöffnung (12, 13) der jeweiligen Messleitung (9, 10) ausbildenden Gasblase (14, 15, 19) gemessen wird.

2. Verfahren nach Anspruch 1, **dadurch gekennzeichnet, dass** die Lanze (1) über mindestens zwei Messleitungen (9, 10) Gas seitlich ausbläst, wobei in Abhängigkeit einer Differenzbildung der gemessenen Innendrücke der Gasblasen (14, 15, 19) Reaktionsdaten zum Reaktionsablauf bestimmt werden.

3. Verfahren nach Anspruch 2, **dadurch gekennzeichnet, dass** auf gleichen Lanzenhöhen ausmündende Messleitungen (9, 10) voneinander unterschiedlich große Gasblasen (14, 19) ausbilden.

4. Verfahren nach Anspruch 2, **dadurch gekennzeichnet, dass** auf unterschiedlichen Lanzenhöhen ausmündende Messleitungen (9, 10) gleich große Gasblasen (14, 15) ausbilden.

5. Verfahren nach Anspruch 2, **dadurch gekennzeichnet, dass** auf unterschiedlichen Lanzenhöhen ausmündende Messleitungen (9, 10) unterschiedlich große Gasblasen (14, 15, 19) ausbilden.

6. Verfahren nach einem der Ansprüche 2 bis 5, **dadurch gekennzeichnet, dass** sich die Messleitungen (9, 10) an gegenüberliegenden Seiten (21, 22) am Lanzenkörper (11), insbesondere am Lanzenkörper (11) diametral gegenüberliegend, Gas ausblasen.

7. Verfahren nach einem der Ansprüche 1 bis 6, **dadurch gekennzeichnet, dass** die Messleitung bzw. die Messleitungen (9, 10) von dem die Lanze kühlenden Kühlmedium mitgekühlt wird bzw. werden.

8. Aufblasverfahren, bei dem dessen Reaktionsablauf anhand von Reaktionsdaten gesteuert oder geregelt wird, die mithilfe eines Verfahrens nach einem der Ansprüche 1 bis 7 bestimmt werden.

9. Lanze zum Aufblasen eines Reaktionsgases (4) auf eine in einem metallurgischen Gefäß (2) befindliche metallische Schmelze (3) mit einem Lanzenkörper (11), der einen wenigstens teilweise gekühlten Lanzenmantel (5) aufweist, mit einer in mindestens einer Öffnung (8) des Lanzenmantels (5) ausmündenden Gasführung (7) für das Reaktionsgas (4), und mit mindestens eine im Lanzenkörper (11) vorgesehene Messleitung (9, 10), die in wenigstens einer Mündungsöffnung (12, 13) am Lanzenmantel (5) des Lanzenkörpers (11) endet und ein gegenüber dem Reaktionsgas (4) getrennt geführtes Gas ausbläst, **dadurch gekennzeichnet, dass** die Messleitung (9, 10) in mindestens einer Mündungsöffnung (12, 13) seitlich am Lanzenmantel (5) des Lanzenkörpers (11) endet und zur Erzeugung mindestens einer Gasblase (14, 15, 19) ausgebildet ist.

10. Lanze nach Anspruch 9, **dadurch gekennzeichnet, dass** eine Messeinrichtung (20) vorgesehen ist, die einen Sensor (17, 18) zur Erfassung von Messdaten aufweist, der mit der Messleitung (9) zur Erfassung von, vom Innendruck der Gasblase (14, 15, 19) abhängigen Messdaten verbunden ist.

11. Lanze nach Anspruch 9 oder 10, **dadurch gekennzeichnet, dass** der Lanzenkörper (11) mindestens zwei Messleitungen (9, 10) aufweist, die seitlich am Lanzenkörper (11) ausmünden.

12. Lanze nach Anspruch 11, **dadurch gekennzeichnet, dass** die Mündungsöffnungen (12, 13) der Messleitungen (9, 10) unterschiedliche Mündungsquerschnitte aufweisen.

13. Lanze nach Anspruch 11 oder 12, **dadurch gekennzeichnet, dass** die Mündungsöffnungen (12, 13) der Messleitungen (9, 10) auf unterschiedlicher Höhe am Lanzenmantel (5) seitlich ausmünden.

14. Lanze nach Anspruch 11, 12 oder 13, **dadurch gekennzeichnet, dass** die Mündungsöffnungen (12, 13) der Messleitungen (9, 10) an gegenüberliegenden Seiten (21, 22) am Lanzenkörper (11), insbesondere am Lanzenkörper (11) diametral gegenüberliegend, ausmünden.

15. Lanze nach einem der Ansprüche 9 bis 14, **dadurch gekennzeichnet, dass** die Messleitung (9, 10) mit dem gekühlten Lanzenmantel (5) des Lanzenkörpers (11) zumindest bereichsweise thermisch leitend verbunden ist.

## Claims

1. Method for the determination of reaction data of a reaction sequence, in which a reaction gas (4) is top-blown by means of at least one lance (1) onto a metallic melt (3) in a metallurgical vessel (2) and simultaneously measured data are recorded, as a function of which reaction data about the reaction sequence are determined, wherein the lance (1) for the recording of measured data blows out a gas being conveyed separately from the reaction gas (4) via at least one outlet opening (12, 13) of at least one measuring line (9, 10), **characterized in that** the lance (1) for the recording of measured data blows out the gas being conveyed separately from the reaction gas (4) laterally via at least one outlet opening (12, 13) of at least one measuring line (9, 10), wherein the internal pressure of at least one gas bubble (14, 15, 19) formed at this outlet opening (12, 13) of the respective measuring line (9, 10) is measured.

2. Method according to claim 1, **characterized in that** the lance (1) blows gas out laterally via at least two measuring lines (9, 10), wherein reaction data about the reaction sequence are determined as a function of an establishment of the difference between the measured internal pressures of the gas bubbles (14, 15, 19).

3. Method according to claim 2, **characterized in that** measuring lines (9, 10) discharging at the same lance height form gas bubbles (14, 19) differing in size from one another.

4. Method according to claim 2, **characterized in that** measuring lines (9, 10) discharging at different lance heights form equally large gas bubbles (14, 15).

5. Method according to claim 2, **characterized in that** measuring lines (9, 10) discharging at different lance heights form gas bubbles (14, 15, 19) of different size.

6. Method according to one of claims 2 to 5, **characterized in that** the measuring lines (9, 10) blow out gas on opposite sides (21, 22) on the lance body (11), especially diametrically oppositely on the lance body (11).

7. Method according to one of claims 1 to 6, **characterized in that** measuring line or the measuring lines (9, 10) is or are simultaneously cooled by the cooling medium that cools the lance.

8. Top-blowing process, in which its reaction sequence is controllable or adjustable on the basis of reaction that are determined by means of a method according to one of claims 1 to 7.

9. Lance for top-blowing a reaction gas (4) onto a metallic melt (3) disposed in a metallurgical vessel (2), with a lance body (11), which has an at least partly cooled lance jacket (5), with a gas supply (7) for the reaction gas (4) discharging in at least one opening (8) of the lance jacket (5), and with at least one measuring line (9, 10), which is provided in the lance body (11) and which ends in at least one outlet opening (12, 13) on the lance jacket (5) of the lance body (11) and blows out a gas being conveyed separately from the reaction gas (4), **characterized in that** the measuring line (9, 10) ends in at least one outlet opening (12, 13) laterally at the lance jacket (5) of the lance body (11) and is formed for the generation of at least one gas bubble (14, 15, 19).

10. Lance according to claim 9, **characterized in that** a measuring device (20) is provided that contains, for the recording of measured data, a sensor (17, 18), which is in communication with the measuring line (9) for the recording of measured data dependent on the internal pressure of the gas bubble (14, 15, 19).

11. Lance according to claim 9 or 10, **characterized in that** the lance body (11) contains at least two measuring lines (9, 10), which discharge laterally on the lance body (11).

12. Lance according to claim 11, **characterized in that** the outlet openings (12, 13) of the measuring lines (9, 10) have different outlet cross sections.

13. Lance according to claim 11 or 12, **characterized in that** the outlet openings (12, 13) of the measuring lines (9, 10) discharge laterally at different heights on the lance jacket (5) .

14. Lance according to claim 11, 12 or 13, **characterized in that** the outlet openings (12, 13) of the measuring lines (9, 10) discharge on opposite sides (21, 22) on the lance body (11), especially diametrically oppositely on the lance body (11).

15. Lance according to one of claims 9 to 14, **characterized in that** the measuring line (9, 10) is in thermally conducting communication, at least zonally, with the cooled lance jacket (5) of the lance body (11).

## Revendications

1. Procédé pour déterminer des données de réaction renseignant sur la progression d'une réaction, dans lequel un gaz de réaction (4) est soufflé à l'aide d'au moins une lance (1) sur un métal liquide (3) dans un conteneur métallurgique (2) pendant que des données de mesure sont détectées, en fonction desquelles sont déterminées des données de réaction renseignant sur la progression de la réaction, la lance (1) destinée à détecter des données de mesure évacuant par soufflage par au moins un trou d'embouchure (12, 13) d'au moins une conduite de mesure (9, 10) un gaz en alimentation séparée, par rapport au gaz de réaction (4), **caractérisé en ce que** la lance (1) destinée à détecter des données de mesure évacue par soufflage latéral par au moins un trou d'embouchure (12, 13) d'au moins une conduite de mesure (9, 10), le gaz en alimentation séparée, par rapport au gaz de réaction (4), à partir duquel est mesurée la pression interne d'au moins une des bulles de gaz (14, 15, 19) se formant audit trou d'embouchure (12, 13) de chacune des conduites de mesure (9, 10).

2. Procédé selon la revendication 1, **caractérisé en ce que** la lance (1) évacue du gaz par soufflage latéral par au moins deux conduites de mesure (9, 10), des données de réaction renseignant sur la progression de la réaction étant déterminées en fonction d'une différence calculée entre les pressions internes mesurées des bulles de gaz (14, 15, 19).

3. Procédé selon la revendication 2, **caractérisé en ce que** des conduites de mesure (9, 10) débouchant à des hauteurs de lance identiques forment des bulles de gaz (14, 19) de tailles différentes les unes par rapport aux autres.

4. Procédé selon la revendication 2, **caractérisé en ce que** des conduites de mesure (9, 10) débouchant à des hauteurs de lance différentes forment des bulles de gaz (14, 15) de tailles identiques.

5. Procédé selon la revendication 2, **caractérisé en ce que** des conduites de mesure (9, 10) débouchant à des hauteurs de lance différentes forment des bulles de gaz (14, 15, 19) de tailles différentes.

6. Procédé selon l'une quelconque des revendications 2 à 5, **caractérisé en ce que** les conduites de mesure (9, 10) évacuent du gaz à des côtés opposés (21, 22) sur le corps de lance (11), notamment diamétralement opposés sur le corps de lance (11).

7. Procédé selon l'une quelconque des revendications 1 à 6, **caractérisée en ce que** la conduite de mesure ou les conduites de mesure (9, 10) est ou sont refroidie(s) en même temps par un agent réfrigérant servant à refroidir la lance.

8. Procédé de soufflage, la progression de la réaction dudit procédé étant commandée ou régulée à partir de données de réaction déterminées à l'aide d'un procédé selon l'une quelconque des revendications 1 à 7.

9. Lance destinée au soufflage d'un gaz de réaction (4) sur un métal liquide (3) se trouvant dans un conteneur métallurgique (2), munie d'un corps de lance (11) qui présente une gaine de lance (5) refroidie au moins partiellement, avec une alimentation en gaz (7) pour le gaz de réaction (4) débouchant dans au moins une ouverture (8) de la gaine de lance (5), et avec au moins, prévue dans le corps de lance (11), une conduite de mesure (9, 10) qui se termine dans au moins un trou d'embouchure (12, 13) sur la gaine de lance (5) du corps de lance (11) et qui évacue par soufflage un gaz en alimentation séparée par rapport au gaz de réaction (4), **caractérisée en ce que** la conduite de mesure (9, 10) se termine dans au moins un trou d'embouchure (12, 13) latéralement sur la gaine de lance (5) du corps de lance (11) et qu'elle est conçue pour générer au moins une bulle de gaz (14, 15, 19).

10. Lance selon la revendication 9, **caractérisée en ce qu'**est prévu un dispositif de mesure (20) qui présente un capteur (17, 18) de détection de données de mesure, lequel est relié à la conduite de mesure (9) pour détecter des données de mesure dépendant de la pression interne des bulles de gaz (14, 15, 19).

11. Lance selon la revendication 9 ou 10, **caractérisée en ce que** le corps de lance (11) présente au moins deux conduites de mesure (9, 10) qui débouchent latéralement sur le corps de lance (11).

12. Lance selon la revendication 11, **caractérisée en ce que** les trous d'embouchure (12, 13) des conduites de mesure (9, 10) présentent des sections d'embouchure différentes.

13. Lance selon la revendication 11 ou 12, **caractérisée en ce que** les trous d'embouchure (12, 13) des conduites de mesure (9, 10) débouchent latéralement à des hauteurs différentes sur la gaine de lance (5).

14. Lance selon l'une quelconque des revendications 11, 12 ou 13, **caractérisée en ce que** les trous d'embouchure (12, 13) des conduites de mesure (9, 10) débouchent sur des côtés opposés (21, 22) sur le corps de lance (11), notamment diamétralement opposés sur le corps de lance (11).

15. Lance selon l'une quelconque des revendications 9 à 14, **caractérisée en ce que** la conduite de mesure (9, 10) est reliée, en termes de conduction thermique, au moins dans certaines zones, à la gaine de lance (5) refroidie du corps de lance (11).
